# FASCICULE DE BREVET EUROPEEN

(11) **EP 1 021 621 B1**
(45) Date de publication et mention de la délivrance du brevet: **13.08.2003**
(21) Numéro de dépôt: 98942804.0
(22) Date de dépôt: 01.09.1998
(51) Int. Cl.: D21H 19/82

(54) **PAPIER OU CARTON A IMPRIMABILITE AMELIOREE**
PAPIER ODER PAPPE MIT VERBESSERTER DRUCKFÄHIGKEIT
PAPER OR CARDBOARD WITH IMPROVED PRINTABILITY

(30) Priorité: 10.09.1997 FR 9711455
(43) Date de publication de la demande: 26.07.2000
(73) Titulaire: Ahlstrom Paper Research and Competence Center, 38780 Pont-Evêque (FR)
(72) Inventeur: GIRARD, Pierre, F-38330 Saint-Ismier (FR); ESCAFFRE, Pascale, F-38260 La Côte Saint André (FR); ROUSSET, Eric, F-38780 Estrablin (FR)
(74) Mandataire: Vuillermoz, Bruno
(86) Numéro de dépôt international: FR9801872
(87) Numéro de publication internationale: WO99013156

(56) Documents cités:
- EP-A- 0 337 771
- EP-A- 0 634 283
- FR-A- 1 449 148
- US-A- 4 440 827

## Description

L'invention concerne un papier ou carton à imprimabilité améliorée, destiné à être imprimé par héliogravure ou flexogravure. Elle se rapporte également au procédé de fabrication d'un tel papier ou carton.

L'héliogravure et la flexogravure sont des techniques d'impression bien connues de l'homme du métier.

Pour l'essentiel, l'héliogravure consiste à presser le papier à imprimer sur un cylindre gravé dont la surface est constituée d'une multitude d'alvéoles d'une taille d'environ 30 à 100 micromètres remplies d'encre fluide. Du fait de sa fluidité, la surface de l'encre forme à l'intérieur de chaque alvéole un ménisque, lequel lors de la rotation du cylindre tend à se déformer et ainsi diminuer le contact entre l'encre et le papier à imprimer.

La flexogravure est un procédé qui s'appuie sur les mêmes principes que ceux de l'héliogravure, mis à part le fait que les éléments imprimants plutôt que d'être en creux sont en relief. De même que précédemment, la qualité de l'impression dépend du contact intime entre l'encre et le papier.

Par ailleurs, il est connu que pour certains papiers « techniques », notamment ceux dont la composition du couchage comporte une forte proportion d'agents liants ainsi que des adjuvants spécifiques, de même que pour les cartons couchés, il est souvent difficile d'obtenir une bonne imprimabilité lors de l'impression par procédé héliogravure ou flexogravure.

Le problème posé est donc celui d'améliorer l'imprimabilité des papiers imprimés par technique d'héliogravure ou flexogravure en cherchant à améliorer le contact de l'encre avec le papier.

Pour améliorer l'imprimabilité on a recours à deux techniques différentes :
- augmenter le poids de couche que ce soit dans le cas d'un papier monocouche ou dans le cas d'un papier double couche,
- améliorer l'état de surface du papier monocouche ou double couche par une action mécanique de calandrage, c'est à dire par passage sous pression de la feuille couchée entre des rouleaux métalliques chauffés et des rouleaux élastiques, ce qui correspond à une opération de supercalandrage
ou « softcalandrage ».

La technique consistant à augmenter le poids de la couche n'est pas satisfaisante dans la mesure où elle est inapplicable pour obtenir des papiers de faible grammage, par exemple de l'ordre de 40 à 45 g/m², dont les caractéristiques mécaniques seraient trop affectées par l'augmentation du poids de la couche au détriment de la masse fibreuse. En outre, l'augmentation du grammage du papier ou de sa couche conduit inévitablement à une dégradation de la résistance aux plis.

De plus et surtout, même si on améliore l'imprimabilité, celle ci reste toutefois insuffisante et l'augmentation du poids du couchage dans le premier cas ou l'opération de calandrage dans le second cas, conduit inéluctablement à diminuer la porosité et donc à fermer le papier à l'air, alors qu'une porosité la plus élevée possible est indispensable pour certains papiers techniques, tels que des papiers techniques complexés avec des matériaux barrière. Par « matériaux barrière », on désigne des matériaux formant notamment barrière aux graisses, aux gaz, à l'eau et la vapeur d'eau, tels que des films du type polyéthylène, polypropylène, polyester ou aluminium, par exemple des assemblages papier - aluminium - polyéthylène.

En effet, ces complexes utilisés dans l'emballage, sont généralement soumis à des opérations de thermoscellage, par exemple thermoscellage polyéthylène - polyéthylène dans le cas susmentionné, engendrant souvent l'apparition d'un phénomène de cloquage. Plus précisément, l'opération de thermoscellage peut entraîner la formation de cloques ou de bulles d'air dues à la vaporisation de l'eau contenue dans le papier, le solvant ou la colle. Dans le cas d'un papier de faible porosité, les vapeurs formées ne peuvent s'échapper à travers celui-ci, entraînant alors la séparation du support papier du revêtement barrière dans les zones de soudure.

Pour éviter le phénomène de cloquage, on est conduit à utiliser un papier monocouche, c'est-à-dire un support enduit d'une couche à base de pigments, lequel présente une bonne porosité par rapport notamment à un papier double couche. Même si la porosité est satisfaisante, et que le papier présente par conséquent une bonne résistance au cloquage dans les zones éventuellement thermoscellées, son imprimabilité reste malheureusement insuffisante.

On se trouve donc confronté à deux problèmes principaux qui sont celui de l'amélioration de l'imprimabilité d'une part, et celui du maintien de la valeur de la porosité d'un papier monocouche d'autre part, et pour lesquels les solutions proposées à ce jour ont un effet radicalement opposé puisqu'on a recours à une augmentation du poids du couchage dans le premier cas et à da densification dans le second cas, ces deux solutions entraînant une réduction de la porosité.

En d'autres termes, aucune des techniques ci-avant décrites, que ce soit l'augmentation du poids de la couche, le double couchage ou le calandrage d'un papier monocouche ou double couche, ne permet d'améliorer l'imprimabilité du papier sans en réduire considérablement la porosité.

On a également proposé dans le document EP-A-0 337 771 un papier destiné à être imprimé par flexogravure. Ce document décrit dans son exemple 1 un support enduit de deux couches, respectivement une première couche à base de bentonite et une seconde couche consistant en une solution aqueuse de kaolin et de copolymère acrylique. Comme le montrent les résultats, le pourcentage de points manquants reste relativement élevé (de l'ordre de 5 %) de sorte que l'imprimabilité ne peut être considérée comme satisfaisante.

Par ailleurs, le document FR-A-1 449 148 décrit un papier d'impression recouvert d'une double couche d'un enduit léger dans lequel la première couche est constituée d'une bouillie aqueuse contenant 15 à 50 % de blanc satin. De plus, il est indiqué que le papier est nécessairement calandré après l'enduction de la première couche, ce qui conduit à augmenter le nombre d'étapes nécessaires au procédé de fabrication.

Parallèlement, on cherche pour certaines applications à diminuer le grammage du produit obtenu sans pour autant en dégrader les caractéristiques mécaniques et ce, notamment dans un souci d'économie. Néanmoins, dans certains cas, on cherche à diminuer uniquement le grammage du couchage de manière à pouvoir reporter ce gain de poids au sein du support fibreux, permettant ainsi de renforcer les propriétés mécaniques du produit final.

L'invention a donc pour objet un nouveau type de papier destiné à être imprimé par héliogravure ou flexogravure, permettant de résoudre l'ensemble des problèmes résumés précédemment et notamment :
- l'amélioration de l'imprimabilité en cherchant à augmenter le contact entre l'encre et le support papier,
- le maintien de la porosité, en particulier dans le cas des papiers techniques complexés,
- et éventuellement la diminution du grammage du produit obtenu sans dégradation de ses propriétés mécaniques.

Pour résoudre le problème relatif à l'imprimabilité, l'invention propose un papier ou carton à imprimabilité améliorée, destiné à être imprimé par héliogravure ou flexogravure, constitué d'un support fibreux enduit d'au moins une couche traditionnelle de surface.

Ce papier ou carton se caractérise en ce qu'il comporte entre le support fibreux et la couche traditionnelle de surface, une couche d'une composition à base de pigments spécifiques destinée à améliorer le contact de la couche traditionnelle de surface avec l'encre d'impression, ladite composition d'une part, comprenant au moins un pigment choisi dans le groupe comprenant la silice, le carbonate de calcium précipité (PCC), seuls ou en mélange, et d'autre part étant déposée sur le support fibreux à raison de 1-5 grammes par mètre carré (1-5 g/m²).

Dans la suite de la description et dans les revendications, par « couche traditionnelle de surface », on désigne une couche de surface comprenant des pigments, des agents liants et des adjuvants, présentant des caractéristiques adaptées à l'application ultérieure, par exemple, papier pour emballage, papier d'impression pour l'édition, papier pour complexage, carton etc... Les compositions de ces couches traditionnelles de surface dépendent donc de l'application envisagée et sont parfaitement connues de l'homme du métier.

En d'autres termes, l'invention consiste à intercaler entre le support fibreux et la couche traditionnelle de surface, que ce soit dans le cas d'un papier ou d'un carton monocouche ou double couche, une masse très faible d'une composition comprenant des pigments présentant des propriétés spécifiques, qui, en permettant d'améliorer la surface de la couche traditionnelle, permet d'améliorer de façon spectaculaire l'imprimabilité, grâce à un contact plus régulier avec l'encre d'impression. Parallèlement, le dépôt de la composition de l'invention sur le support fibreux permet d'obtenir une microporosité superficielle très régulière dudit support, ce qui contribue également à l'amélioration de l'imprimabilité.

En outre, le gain d'imprimabilité est tellement élevé et ce, même lorsque la composition de l'invention est déposée sur le support fibreux à un taux très faible, qu'il est possible de diminuer le dépôt de la couche traditionnelle, ce qui conduit non seulement à réduire nettement le grammage du papier final (d'environ 10%), sans pour autant en dégrader les propriétés mécaniques, mais également selon le choix du pigment, à maintenir ou même améliorer la porosité du papier ou carton obtenu. La réduction du dépôt au niveau de la couche traditionnelle permet aussi, dans certains cas, d'augmenter d'autant la masse du support fibreux conférant au papier final de meilleures propriétés mécaniques, notamment en termes de rigidité, de résistance à la traction, à l'éclatement et la déchirure.

Parallèlement, le fait de diminuer le grammage de couche permet de réduire le phénomène de cassure au plis qui apparaît d'autant plus que le poids de la couche est élevé.

Dans le procédé de l'invention, on peut utiliser tout type de silice choisie dans le groupe comprenant les silices colloïdales, précipitées ou pyrogénées.

On a constaté qu'on obtenait de très bons résultats avec des silices précipitées de surface spécifique comprise entre 150 et 250 m²/g.

De même, parmi les carbonates de calcium précipités, on peut utiliser un carbonate de calcium colloïdal de surface spécifique, avantageusement comprise entre 25 et 40 m²/g.

Lorsqu'on dépose ladite composition à base de pigments spécifiques à un taux supérieur à 5g/m², l'effet bénéfique sur l'imprimabilité existe mais le produit devient moins intéressant non seulement économiquement mais également du fait qu'on augmente le poids du papier fini.

La composition à base de pigments spécifiques est déposée sur le support fibreux à raison de 1-5 gramme par mètre carré, avantageusement entre 1 et 3 g/m².

Pour une valeur de dépôt inférieure à 1 g/m², on constate en effet que la caractéristique d'imprimabilité n'est pas améliorée de manière significative.

Pour permettre à la fois l'amélioration de l'imprimabilité et le maintien ou l'amélioration de la porosité par rapport à un papier monocouche, la composition à base de pigments spécifiques est exclusivement constituée de silice.

On a observé, en effet, que de façon très surprenante, ce pigment permettait d'atteindre simultanément les deux objectifs et ce, toujours pour une valeur très faible de dépôt sur le support fibreux. Il s'ensuit que le papier obtenu convient en particulier pour la fabrication de papier technique soumis à des opérations de thermoscellage après complexage et donc susceptible de développer des phénomènes de cloquage.

L'invention concerne également un procédé pour la fabrication d'un papier ou d'un carton, destiné à être imprimé par héliogravure ou flexogravure, qui consiste :
- à réaliser un support fibreux à partir d'une suspension papetière,
- puis à enduire le support d'au moins une couche traditionnelle de surface,
- à sécher le papier ou le carton ainsi formé,
- et enfin à calandrer le papier ou le carton obtenu.

Ce procédé se caractérise en ce que :
- on dépose préalablement sur le support fibreux au plus cinq grammes par mètre carré (5 g/m²) d'une composition à base de pigments spécifiques choisis dans le groupe comprenant la silice, le carbonate de calcium précipité, seuls ou en mélange ;
- puis on sèche le support fibreux ainsi recouvert avant enduction de la couche traditionnelle de surface.

Comme déjà dit, le dépôt de la composition sur le support fibreux est effectuée à raison de 1-5 g/m², ce qui permet également de réduire le grammage de la couche traditionnelle et ainsi d'augmenter la masse du support fibreux de base, et donc les propriétés mécaniques du papier ou du carton final.

Avantageusement, le dépôt de la composition à base de pigments spécifiques sur le support fibreux est réalisé par enduction, l'ensemble des opérations étant réalisé dans les conditions habituelles de fabrication de papier ou carton couché.

En outre, le dépôt de la composition à base de pigments spécifiques sur le support fibreux, puis l'enduction de la couche traditionnelle sont réalisés à l'aide d'une coucheuse classique ou d'une presse encolleuse encore appelée « size press », ou d'une presse encolleuse avec prédosage encore dénommée « metering size press (MSP) ». Les deux enductions sont réalisées soit sur machine à papier, soit hors machine.

Concernant l'étape de calandrage, elle est effectuée au moyen d'une softcalandre ou d'une supercalandre dans les conditions traditionnelles de fabrication de papier couché.

Comme déjà dit, on constate une très nette amélioration de la qualité de la surface de la couche traditionnelle sur les supports préalablement couchés avec la composition à base de pigments spécifiques de l'invention.

En particulier, le procédé ci-avant décrit permet de fabriquer un papier de faible grammage qui possède d'excellentes propriétés d'impression en héliogravure
ou flexogravure.

Néanmoins, on peut également fabriquer selon le même procédé un papier de grammage élevé présentant une bonne porosité ainsi qu'une bonne imprimabilité, quelque soit le procédé d'impression.

La manière de réaliser l'invention et les avantages qui en découlent ressortiront mieux des différents exemples de réalisation suivants.

### Exemple comparatif 1

On compare les valeurs d'imprimabilité et de porosité d'un papier monocouche, d'un papier double couche et d'un papier caractéristique de l'invention, dont le point commun est de présenter une couche traditionnelle de surface de même composition.

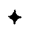 **Composition et préparation du papier monocouche** (comparatif)

On prépare une sauce d'enduction dont la composition, donnée en parties pondérales sec/sec, figure dans le tableau ci-après :

| | | |
|---|---|---|
| Pigments | AMAZON 90 (kaolin) ¹ BLANC SATIN ² | 85 15 |
| Agent liant | ACRONAL A 360 D ³ | 14 |
| Agent épaississant | RHEOCOAT 35 ⁴ | 0,4 |
| Agent insolubilisant | URECOLL SU ⁵ | 2,3 |
| Agent dispersant | GX ⁶ | 0,2 |
| Agent lubrifiant | CECAVON CA 350 ⁷ | 1 |

| | | |
|---|---|---|
| 1 : marque déposée, produit commercialisé par KAOLIN D'ARVOR | | |
| 2 : produit commercialisé par SUPRASMIT | | |
| 3, 5 : marques déposées, produits commercialisés par BASF | | |
| 4, 6 : marque déposée, produits commercialisés par COATEX | | |
| 7 : marque déposée, produit commercialisé par ELF ATOCHEM | | |

On dépose 12,1 g/m² de la couche ainsi préparée sur un support fibreux préalablement fabriqué, par enduction au moyen d'une coucheuse du type lame métallique. On sèche et on calandre le papier obtenu.

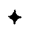 **Composition et préparation du papier double couche** (comparatif)

On prépare une première sauce d'enduction, correspondant à la première couche, dont la composition, donnée en parties pondérales sec/sec, figure dans le tableau ci-après :

| | | |
|---|---|---|
| Pigment | OMYALITE 90 (Carbonate de Calcium naturel) ⁸ | 100 |
| Agent liant | ACRONAL A 360 D | 13 |
| Agent épaississant | RHEOCOAT 35 | 0,4 |
| Agent insolubilisant | URECOLL SU | 0,8 |
| Agent dispersant | GX | 0,1 |

| | | |
|---|---|---|
| 8 : marque déposée, produit commercialisé par OMYA | | |

On dépose sur machine à papier à l'aide d'une coucheuse à barre doseuse ou à lame métallique, 6,3 g/m² de la sauce d'enduction ainsi préparée sur un support fibreux préalablement fabriqué.

Après séchage de cette couche, on enduit en ligne une seconde sauce d'enduction, dont la composition correspond à celle utilisée dans le papier monocouche ci-avant.

On dépose 8,1 g/m² de cette sauce d'enduction sur la première couche, à l'aide d'une coucheuse à lame métallique.

On sèche et on calandre le papier obtenu dans les mêmes conditions que précédemment.

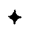 **Composition et préparation du papier de l'invention**

On prépare la composition correspondant au papier de l'invention, dont les caractéristiques figurent dans le tableau ci-après :

| | | |
|---|---|---|
| Pigment | SK 300 DS ⁹ (Silice précipitée) | 100 |
| Agent liant | ACRONAL A 360 D | 60 |
| Agent insolubilisant | URECOLL SU | 1 |
| Agent dispersant | GX | 0,1 |

| | | |
|---|---|---|
| 9 :marque déposée, produit commercialisé par DEGUSSA | | |

La surface spécifique de la silice utilisée est d'environ 200 m²/g.

On dépose à l'aide d'une coucheuse à barre doseuse, 2,9 g/m² de la composition ainsi préparée sur un support fibreux.

Après séchage, on dépose 8 g/m² d'une sauce d'enduction dont la composition est identique à celle du papier monocouche précédemment fabriqué.

On sèche et on calandre ensuite le papier obtenu dans les mêmes conditions que précédemment.

On a regroupé dans le tableau ci après les résultats d'imprimabilité et de porosité des différents papiers ainsi fabriqués.

L'évaluation de l'imprimabilité est effectuée par la technique de l'Héliotest, qui consiste à mesurer la distance du vingtième point manquant sur l'impression d'une bande de papier, à l'aide d'un appareil connu sous le nom « d'appareil IGT ». Les résultats sont donnés en millimètres.

La mesure de la porosité SCAN est effectuée par la technique de LORENTZEN. Les résultats sont donnés en cm³/ m².s.

| | **Papier monocouche** | **Papier double couche** | **Papier de l'Invention** |
|---|---|---|---|
| **Imprimabilité** **(Héliotest-mm)** | 23 | 18 | 82 |
| **Porosité** | 820 | 630 | 1100 |
| **Résistance au cloquage à 190°C après complexage avec de l'aluminium 18 micromètres** | cloquage | cloquage | pas de cloquage |
| **Grammage du papier couché** **(g/m**^{**2**}**)** | 67 | 69,3 | 65,8 |
| **Poids de couche totale (g/m**^{**2**}**)** | 12,1 | 14,4 | 10,9 |

On observe donc une forte augmentation de l'imprimabilité dans le cas du papier de l'invention. De plus, l'imprimabilité est augmentée et ce, malgré un dépôt de la composition extrêmement faible, de l'ordre de 3g/m². Ce faible dépôt de composition permet de diminuer sensiblement le poids de la couche traditionnelle (10,9 g/m²) et donc d'améliorer nettement la porosité (1100) par rapport à celle d'un papier monocouche traditionnel (820). Il s'ensuit que l'on n'observe pas de cloquage avec le papier de l'invention lorsqu'il s'agit d'un papier complexé, soumis à une opération de thermoscellage.

On note que le papier double couche standard présente une imprimabilité du niveau du papier monocouche malgré un poids de couche total supérieur. Ce résultat n'est pas surprenant dans la mesure où le double couchage standard n'engendre pas toujours une augmentation de l'imprimabilité pour des poids de couche faibles, inférieurs à 16 g/m² du fait de la nécessité de diluer les bains de couchage pour limiter le dépôt de couche.

### Exemple comparatif 2

On compare les valeurs d'imprimabilité et de porosité de trois papiers double couche et trois papiers caractéristiques de l'invention, dont la couche traditionnelle de surface présente trois compositions différentes.

On a représenté dans la tableau suivant la composition donnée en parties pondérales sec/sec, de ces trois couches traditionnelles de surface:

| | | couche de surface 1 | couche de surface 2 | couche de surface 3 |
|---|---|---|---|---|
| Pigment | AMAZON 90 OMYALITE 90 | 88 12 | 50 50 | 100 --- |
| Agent liant | ACRONAL A 360 D | 13 | 13 | 13 |
| Agent épaississant | RHEOCOAT 35 | 0,4 | 0,4 | 0,4 |
| Agent lubrifiant | CECAVON CA 350 | 1 | 1 | 1 |
| Agent insolubilisant | URECOLL SU | 0,7 | 0,7 | 0,7 |
| Agent dispersant | GX | 0,1 | 0,1 | 0,1 |

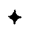 **Composition et préparation du papier double couche** (comparatif)

On prépare une sauce d'enduction dont la composition donnée en parties pondérales sec/sec, figure dans le tableau suivant :

| | | |
|---|---|---|
| Pigments | AMAZON 90 OMYALITE 90 | 34 66 |
| Agents liants | ACRONAL A 360 D ACTISIZE 80 ¹⁰ | 13 11 |
| Agent lubrifiant | CECAVON CA 350 | 0,3 |
| Agent insolubilisant | URECOLL SU | 1 |
| Agent dispersant | GX | 0,1 |

| | | |
|---|---|---|
| 10 : marque déposée, produit commercialisé par ROQUETTE | | |

Sur un support fibreux préalablement préparé, on enduit la couche ainsi préparée à raison de 9 g/m² à l'aide d'une coucheuse à barre doseuse ou lame métallique.

On dépose ensuite 10 g/m² de la couche de surface 1 à l'aide d'une coucheuse type lame métallique.

On fabrique ensuite deux autres papiers double couche avec la couche de surface 2 puis avec la couche de surface 3, toutes deux déposées à raison de 10 g/m² sur la première couche

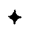 **Composition et préparation du papier de l'invention**

On prépare une sauce d'enduction dont la composition donnée en parties pondérales sec/sec, est :

| | | |
|---|---|---|
| Pigment | SK 300 DS | 100 |
| Agent liant | ACRONAL A 360 D ACTISIZE 80 | 20 20 |
| Agent lubrifiants | CECAVON CA 350 | 0,3 |
| Agent insolubilisant | URECOLL SU | 1 |
| Agent dispersant | GX | 0,1 |

On dépose à l'aide d'une coucheuse type barre doseuse, 3 g/m² de cette sauce d'enduction sur un support fibreux préalablement fabriqué.

Après séchage, on enduit la couche de surface 1.

On répète la même opération avec les couches de surface 2 et 3, toutes trois déposées à raison de 10 g/m² sur la première couche, à l'aide d'une coucheuse à lame métallique.

Après séchage et calandrage dans des conditions identiques, on a évalué les résultats d'imprimabilité par Héliotest, et la porosité SCAN des différents papiers obtenus.

| | **Papier double couche** | | | **Papier de l'invention** | | |
|---|---|---|---|---|---|---|
| | Couche de surface 1 | Couche de surface 2 | Couche de surface 3 | Couche de surface 1 | Couche de surface 2 | Couche de surface 3 |
| lmprimabilité Héliotest | 25 | 37 | 38 | > 110 | >110 | >110 |
| Porosité SCAN | 160 | 150 | 200 | 1100 | 930 | 1500 |
| Poids de couche total (g/m²) | 19 | 19 | 19 | 13 | 13 | 13 |
| grammage du papier final (g/m²) | 95 | 95 | 95 | 89 | 89 | 89 |

On constate donc que le papier de l'invention présente une imprimabilité excellente par rapport à un papier double couche traditionnelle et ce, avec une couche de surface de composition identique. De plus, le poids de couche est nettement inférieur par rapport à un papier double couche, de sorte que le grammage du papier final est réduit d'autant. De même, on note une porosité excellente du papier de l'invention. On obtient également d'excellents résultats d'Héliotest avec le papier de l'invention (>110), et ce quelque soit la composition pigmentaire de la couche de surface. L'invention permet donc de modifier avec une grande souplesse la nature de la composition de la couche de surface, et notamment de choisir des pigments peu chers, ou plus blancs, ou encore favorables au développement du brillant ou de la matité de la surface tout en assurant une bonne imprimabilité.

### Exemple comparatif 3

On compare les valeurs d'imprimabilité et de porosité d'un papier monocouche et d'un papier caractéristique de l'invention, dont la composition recouvrant le support fibreux contient des pigments de nature différente.

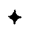 **Composition et préparation du papier monocouche**

On enduit un support fibreux préalablement préparé d'une couche traditionnelle, dont la composition est :

| | | |
|---|---|---|
| Pigments | AMAZON 90 (kaolin) ¹ BLANC SATIN ² | 85 15 |
| Agent liant | ACRONAL A 360 D ³ | 16 |
| Agent épaississant | RHEOCOAT 35 ⁴ | 0,3 |
| Agent lubrifiant | CECAVON CA 350⁷ | 0,9 |

On dépose 8,9 g/m² de cette couche sur le support fibreux à l'aide d'une coucheuse à barre doseuse. Le support est ensuite séché puis calandré.

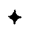 **Composition et préparation du papier de l'invention**

On prépare 3 compositions différentes référencées ci-après A, B, C.

### Composition A

| | | |
|---|---|---|
| pigment | SK 300 DS | 100 |
| Agent liant | ACRONAL A 360 D | 60 |
| Agent insolubilisant | URECOLL SU | 1 |

On dépose 2,2 g/m² de cette composition sur le support fibreux à l'aide d'une coucheuse à barre doseuse.

Après séchage, on enduit ensuite la couche traditionnelle, dont la composition est identique à celle du papier monocouche ci-avant préparée, à raison de 7,8 g/m².

### Composition B

| | | |
|---|---|---|
| Pigment | PCC COLLOIDAL ¹¹ (carbonate de calcium précipité) | 100 |
| Agent liant | ACRONAL A 360 D | 20 |
| Agent insolubilisant | URECOLL SU | 1 |

| | | |
|---|---|---|
| 11 : marque déposée, produit commercialisé par FAXE KALK | | |

On dépose 2,3 g/m2 de cette composition sur le support fibreux base à l'aide d'une coucheuse à barre doseuse.

Après séchage, on enduit la couche traditionnelle préparée pour le papier monocouche, à raison de 7,8 g/m².

### Composition C

| | | |
|---|---|---|
| Pigment | PCC COLLOIDAL SK 300 DS | 50 50 |
| Agent liant | ACRONAL A 360 D | 20 |
| Agent insolubilisant | URECOLL SU | 1 |

On dépose 2,6 g/m² de cette composition sur le support fibreux de base à l'aide d'une coucheuse à barre doseuse.

Après séchage, on enduit la couche traditionnelle, dont la composition est identique à celle du papier monocouche ci-avant préparée, à raison de 6,2 g/m².

On a regroupé dans les tableaux suivants les résultats de l'imprimabilité et de la porosité des différents papiers obtenus.

| | **papier monocouche** | **Papier A** | **Papier B** | **Papier C** |
|---|---|---|---|---|
| **Imprimabilité** **(Héliotest)** | 18 | 94 | 49 | 63 |
| **Porosité** | 760 | 570 | 480 | 290 |
| **Poids de couche total (g/m**^{**2**}**)** | 8,9 | 10 | 10,2 | 8,8 |
| **Grammage papier (g/m**^{**2**}**)** | 46 | 46,5 | 46,5 | 70 |

On constate donc une amélioration importante de l'imprimabilité du papier de l'invention par rapport à un papier monocouche.

Suivant le choix des pigments de la composition de l'invention, il est possible soit de favoriser le gain d'imprimabilité (papier A, C), soit de favoriser le maintien élevé de la porosité (papier A). Le papier A permet de réaliser ces deux objectifs et peut être utilisé notamment pour la fabrication de papiers techniques. Dans les cas, où on ne se préoccupe pas de la porosité, on choisira les autres pigments.

Il ressort donc de l'exposé que l'invention présente un grand nombre d'avantages.

En effet, elle propose un papier pour héliogravure ou flexogravure qui présente une imprimabilité fortement améliorée par rapport aux papiers monocouche et double couche, connus jusqu'alors, tout en conservant une porosité proche de celle d'un papier monocouche.

De la sorte, cette invention peut être utilisée dans de nombreuses applications, notamment lorsqu'il est nécessaire d'adjoindre un matériau barrière, tel que de l'aluminium, le polyéthylène, le polypropylène ou le polyester à un papier couché, sans risquer le phénomène de cloquage lors du thermoscellage.

De même, le faible grammage de la composition incorporée entre le support fibreux et la couche standard traditionnelle permet non seulement de diminuer de façon appréciable le grammage du papier final, mais également dans certains cas, de renforcer d'autant le poids du support fibreux, de sorte à améliorer ses propriétés mécaniques.

De même, le grammage réduit du papier fini permet d'éviter les phénomènes de cassure aux plis.

Par ailleurs, ce papier peut être fabriqué par un procédé mettant en oeuvre des techniques usuelles telles que l'enduction par coucheuse, par size press ou encore metering size press (MSP).

On obtient ainsi des complexes utilisables dans l'emballage alimentaire dont les propriétés d'imprimabilité et de thermoscellage sont fortement améliorées.

## Revendications

1. Papier ou carton constitué d'un support fibreux enduit d'au moins une couche de surface, dont la composition est adaptée à une impression par héliogravure ou flexogravure, et comportant entre le support fibreux et la couche de surface, une couche d'une composition à base de pigments spécifiques, déposée à raison de un à cinq grammes par mètre carré (1 à 5g/m²), **caractérisé en ce que** les pigments spécifiques sont choisis dans le groupe comprenant la silice et le carbonate de calcium précipité (PCC), seuls ou en mélange.

2. Papier ou carton selon la revendication 1, **caractérisé en ce que** les pigments spécifiques sont exclusivement constitués de silice.

3. Papier ou carton selon la revendication 1, **caractérisé en ce que** les pigments spécifiques sont exclusivement constitués de carbonate de calcium précipité.

4. Papier ou carton selon l'une des revendications 1 à 3, **caractérisé en ce que** les pigments spécifiques sont constitués de silice et de carbonate de calcium précipité en proportions égales.

5. Papier ou carton selon l'une des revendications 1 à 3, **caractérisé en ce que** la composition à base de pigments spécifiques est déposée à raison d'au moins 1 gramme par mètre carré (1 g/m2), avantageusement entre un et trois grammes par mètre carré (1 et 3 g/m²).

6. Procédé pour la fabrication d'un papier ou d'un carton, destiné à être imprimé par héliogravure ou flexogravure, qui consiste :
• à réaliser un support fibreux à partir d'une suspension papetière,
• puis à enduire le support d'au moins une couche de surface, dont la composition est adaptée à une impression par héliogravure ou flexogravure ;
• à sécher le papier ou le carton ainsi formé,
• et enfin à calandrer le papier ou le carton obtenu,
**caractérisé en ce que** :
• on dépose préalablement sur le support fibreux entre un et cinq grammes par mètre carré (1 et 5 g/m²) d'une composition à base de pigments spécifiques choisis dans le groupe comprenant la silice et le carbonate de calcium précipité, seuls ou en mélange ;
• puis, on sèche le support fibreux ainsi recouvert avant enduction de la couche traditionnelle de surface.

7. Procédé selon la revendication 6, **caractérisé en ce que** le dépôt de la composition à base de pigments spécifiques sur 1e support fibreux est réalisé par enduction.

8. Procédé selon la revendication 7, caractérisé en ce le dépôt de la composition à base de pigments spécifiques sur le support fibreux, puis l'enduction de la couche traditionnelle sont réalisés à l'aide d'une coucheuse, ou d'une presse encolleuse (size press), ou d'une presse encolleuse avec prédosage (meterig size press (MSP)).

## Patentansprüche

1. Papier oder Karton bestehend aus einem faserigen Träger, der mit mindestens einer Oberflächenschicht beschichtet ist, deren Zusammensetzung für einen Aufdruck mittels Tiefdruck oder Flexodruck ausgelegt ist, und welches zwischen dem faserigen Träger und der Oberflächenschicht eine Schicht aus einer Zusammensetzung auf der Basis spezifischer Pigmente aufweist, die in einer Menge von ein bis fünf Gramm pro Quadratmeter (1 bis 5 g/m²) aufgebracht ist, **dadurch gekennzeichnet, daß** die spezifischen Pigmente aus der Gruppe ausgewählt sind, die Kieselerde und ausgefälltes Kalziumkarbonat (PCC) einzeln oder in Mischung umfaßt.

2. Papier oder Karton nach Anspruch 1, **dadurch gekennzeichnet, daß** die spezifischen Pigmente ausschließlich aus Kieselerde bestehen.

3. Papier oder Karton nach Anspruch 1, **dadurch gekennzeichnet, daß** die spezifischen Pigmente ausschließlich aus ausgefälltem Kalziumkarbonat bestehen.

4. Papier oder Karton nach einem der Ansprüche 1-3, **dadurch gekennzeichnet, daß** die spezifischen Pigmente zu gleichen Teilen aus Kieselerde und ausgefälltem Kalziumkarbonat bestehen.

5. Papier oder Karton nach einem der Ansprüche 1-3, **dadurch gekennzeichnet, daß** die Zusammensetzung auf der Basis spezifischer Pigmente in einer Menge von mindestens ein Gramm pro Quadratmeter (1 g/m²), vorzugsweise zwischen ein und drei Gramm pro Quadratmeter (1 und 3 g/m²) aufgebracht ist.

6. Verfahren zur Herstellung eines Papiers oder Kartons, welches zum Bedrucken mittels Tiefdruck oder Flexodruck bestimmt ist, bestehend aus:
• Herstellen eines faserigen Trägers aus einer Papiersuspension,
• anschließend Beschichten des Trägers mit mindestens einer Oberflächenschicht, deren Zusammensetzung für einen Aufdruck durch Tiefdruck oder Flexodruck ausgelegt ist;
• Trocknen des so gebildeten Papiers oder Kartons,
• schließlich Kalandrieren des erhaltenen Papiers oder Kartons,
**dadurch gekennzeichnet, daß**
• zuvor auf dem faserigen Träger zwischen eins und fünf Gramm pro Quadratmeter (1 und 5 g/m²) einer Zusammensetzung auf der Basis spezifischer Pigmente aufgebracht wird, welche aus der Gruppe umfassend Kieselerde und ausgefälltes Kalziumkarbonat allein oder in Mischung aufgebracht ist;
• der so beschichtete faserige Träger vor Beschichtung mit der herkömmlichen Oberflächenschicht anschließend getrocknet wird.

7. Verfahren nach Anspruch 6, **dadurch gekennzeichnet, daß** das Aufbringen der Zusammensetzung auf der Basis spezifischer Pigmente auf dem faserigen Träger durch Beschichtung erfolgt.

8. Verfahren nach Anspruch 7, **dadurch gekennzeichnet, daß** dieses Aufbringen der Zusammensetzung auf der Basis spezifischer Pigmente auf dem faserigen Träger und die anschließende Beschichtung durch die herkömmliche Schicht mit Hilfe einer Streichmaschine oder einer Leimpresse (Sizepress) oder einer Leimpresse mit Vordosierung (Metering Size Press (MSP)) erfolgt.

## Claims

1. Paper or board consisting of a fibrous medium coated with at least one surface coat, the composition of which is designated for gravure or flexographic printing and including, between the fibrous medium and the surface coat, a coat of a composition based on specific pigments, this coat being deposited in an amount from one to five grams per square metre (1 to 5 g/m²), **characterized in that** the specific pigments are chosen from the group comprising silica and precipitated calcium carbonate (PCC) on their own or as a mixture.

2. Paper or board according to Claim 1, **characterized in that** the specific pigments consist exclusively of silica.

3. Paper or board according to Claim 1, **characterized in that** the specific pigments consist exclusively of precipitated calcium carbonate.

4. Paper or board according to one of Claims 1 to 3, **characterized in that** the specific pigments consist of silica and precipitated calcium carbonate in equal proportions.

5. Paper or board according to one of Claims 1 to 3, **characterized in that** the composition based on specific pigments is deposited in an amount of at least one gram per square metre (1 g/m²), advantageously between one and three grams per square metre (1 and 3 g/m²).

6. Process for the manufacture of a paper or of a board, intended to be printed by gravure or flexographic printing, which consists:
• in producing a fibrous medium from a paper suspension,
• then in coating the medium with at least one surface coat, the composition of which is designated for gravure or flexographic printing,
• in drying the paper or the board thus formed,
**characterized in that**:
• between one and five grams per square metre (1 and 5 g/m²) of a composition based on specific pigments chosen from the group comprising silica and precipitated calcium carbonate, on their own or as a mixture, are deposited beforehand on the fibrous medium;
• and then, the fibrous medium thus covered is dried before it is coated with the conventional surface coat.

7. Process according to Claim 6, **characterized in that** the deposition of the composition based on specific pigments on the fibrous medium is carried out by coating.

8. Process according to Claim 7, **characterized in that** the deposition of the composition based on specific pigments on the fibrous medium followed by the coating of the conventional coat are carried out using a coater, or a size press, or a metering size press (MSP).
